# EUROPEAN PATENT APPLICATION

(11) **EP 0 849 358 A2**
(43) Date of publication of application: **24.06.1998**
(21) Application number: 97890235.1
(22) Date of filing: 25.11.1997
(51) Int. Cl.: C12N 9/02, G01N 33/84, C12Q 1/26, C02F 3/34

(54) **Nitrate detection, measurement, and treatment system**

(30) Priority: 25.11.1996 US 758147
(71) Applicant: Campbell, Wilbur, Dr., Lake Linden, MI 49945 (US); Campbell, Ellen R., Lake Linden, MI 49945 (US)
(72) Inventor: Campbell, Wilbur, Dr., Lake Linden, MI 49945 (US); Campbell, Ellen R., Lake Linden, MI 49945 (US)
(74) Representative: Matschnig, Franz, Dipl.-Ing.

(57) **Abstract**

A plurality of nitrate determination techniques to determine the presence and/or concentration of nitrate containing compounds within water, food, and/or virtually any other target medium. In one embodiment of the invention a home based apparatus is provided which may be used by a homeowner to determine the relative presence and/or concentration of nitrate containing compounds with food and/or with drinking water. In another aspect of the invention, a water treatment system is provided including an annular container and a plurality of filters disposed within the container.

## Description

### BACKGROUND OF THE INVENTION

### 1. Field of the Invention

This invention relates to a method and to an apparatus for determining amounts of nitrate in various mediums and/or substances, such as and without limitation, water, raw or prepared foods such as vegetable juices and baby foods, and to various types of relatively easy to use and relatively inexpensive "home based" testing kits which may be used by individuals to determine the nitrate concentration in these various mediums. Furthermore, this invention also relates, without limitation, to water treatment and/or water purification apparatuses and methodologies, and more particularly, to a method and apparatus to reduce or eliminate nitrate from municipal, or other types of relatively large-scale water distribution systems, and to a method and apparatus for the production of nitrate reductase which may be used by and within the various embodiments of the invention.

### 2. Discussion

Nitrate and nitrate containing compounds or substances (hereinafter the term "nitrate containing compounds" and/or "nitrate containing substances" are interchangeably used to mean and/or to refer to either or both substantially "pure nitrate as well as nitrate containing substances and nitrate containing chemical compounds and/or biological sources) contaminate groundwater in many parts of the United States and in many parts of Europe and/or Asia, causing deformed and or still-born babies and various other types of physical maladies.

For example and without limitation, it is known that relatively high concentrations of nitrate containing compounds in groundwater may cause such maladies as methemoglobinemia in young animals and humans and one or more forms or types of stomach cancer and lymphoma. Accordingly, such nitrate contamination has caused and continues to cause drinking water systems to become polluted and unsafe. These nitrate containing compounds typically enter the ecosystem by means of fertilizers and animal and/or human excretions and/or manure. Hence, a long term solution to the world-wide nitrate pollution problem is recognized to potentially be for the governments of the world to actively police the type of chemicals entering into their own respective ecosystems. However, this long-term solution may not be practical since environmental concerns differ greatly from government to government. Moreover, since many countries are contiguously connected to a number of other countries, nitrate pollution may leach across these various territorial borders, causing inter-country pollution. Obviously, such leaching type pollution may even adversely affect a country which actively polices the pollution occurring solely within its own borders. Moreover, and perhaps more importantly, none of these potential long term solutions impacts or rectifies the present contamination problem. There is therefore a great need for a technique (throughout remainder of this Application, the term "technique" is used to refer to a methodology and/or a system and/or an apparatus), to decrease, partially remove, and/or eliminate the amount of nitrate containing compounds in the environment. There is also a great need for a relatively inexpensive and relatively easy to use technique to allow a homeowner or other type of individual to quickly and accurately determine the amount of and/or the presence of nitrate containing compounds within drinking water. There is also a great need, in order to create the desired techniques of this invention, for a method and/or an apparatus to produce nitrate reductase in a relatively inexpensive, and relatively reliable manner, and which produces the nitrate reductase in a relatively pure form. As will be seen below, Applicant's invention addresses these needs in a novel and non-obvious manner.

While many systems currently exist for removing nitrate containing compounds from the ecosystem, they each suffer from several drawbacks. For example, a class of physico-chemical techniques are known (e.g. electrodialysis, reverse osmosis, and ionexchange methodologies) which remove such nitrate containing compounds from one part of the environment and place it back in another part of environment at some later time period. These techniques do not degrade or chemically alter the "treated" nitrate containing compounds, but merely process them or take them out of a "given" solution, store the extracted material, and place the extraction back into the environment at a much higher concentration. This "shell game" approach obviously fails to totally eliminate any of these nitrate containing compounds from the overall environment of the world and fails to achieve any of the afore-described long-term elimination objectives. These prior techniques are not viewed as being viable.

In contrast to these afore-described physico-chemical techniques, there is also used and known a second general class of prior "techniques" in which one or more bacterial cultures are employed to chemically reduce the nitrate in these target compounds to relatively environmentally biologically and chemically "friendly" nitrogen gas. While these bacterial techniques effectively reduce the overall amount or level of nitrogen, they are relatively slow, are relatively complicated to use since various levels of additional substances must be added to the target medium before the bacterial technique is employed, and require a relatively great amount of effort to obtain the necessary amount of bacterial cultures. This technique is therefore regarded as highly inefficient. For these reasons, these prior techniques are not viewed as being viable. In sum, there exists a great need to provide a method, technique, and/or apparatus for effectively and relatively quickly removing nitrate containing compounds from a target substance or an environment in a manner which overcomes many, if not all, of these afore-described difficulties of the prior art.

Many other types of prior techniques and/or systems also exist to determine the amount of nitrate present in a variety of substances or compounds. For instance, colorimetric determination of nitrate has been done by analyzing products of reagents which are oxidized by nitrate. Typically, such techniques employ the known brucine method. These prior techniques, while somewhat effective, produce highly variable results and allow multiple and contradictory inferences to be drawn. Additionally, other methods and techniques are known and used in which a direct determination of nitrate concentration is done by the use of polarography, direct potentiometry with nitrate ion-selective electrodes, and ultraviolet absorption. While all of these prior methods and techniques provide some indication of nitrate concentration, they each suffer from many drawbacks. That is, by way of example and without limitation, these prior techniques do not yield relatively highly reliable concentration results. Perhaps the most widely used nitrate concentration determination technique is that in which a Copper-Cadmium reagent is used to reduce nitrate in water to nitrite. While this technique provides somewhat reliable concentration results, it too suffers from many drawbacks. For example, and without limitation, the reagent may become inactive by contaminating substances. Moreover, the reagent is known to be relatively environmentally "unfriendly" since it comprises and contains "heavy metal". Disposal of the reagent after the process is completed is therefore a problem. Lastly, there is also known a prior class of techniques which use nitrate reductase to determine the concentration of nitrate by reduction. While these prior techniques replace the relatively environmentally "unfriendly" heavy metal reagent with nitrate reductase, they do not provide consistently reliable concentration results in a cost effective manner and do so in a manner which provides only an indication of concentration levels and does not address or solve the problem of actually removing the determined concentration of the nitrates from the environment. Moreover, these prior techniques are relatively complicated and expensive.

Accordingly, there is a need to provide one or more techniques which overcome the disadvantages of the prior art and which are capable of determining the concentration of nitrate in various substances or an environment without the use of environmentally "unfriendly" reagents, in a relatively cost effective and/or inexpensive manner, with a relatively high degree of accuracy, and in a manner which is relatively easy to use, even to a "lay" or non-scientific person. There is a further need to provide a technique for the treatment and/or purification of nitrate containing water which may be used on a relatively large scale, such as, and without limitation, by municipalities.

### SUMMARY OF THE INVENTION

While a number of "objects of the invention" are set forth below, it should be realized that the invention is not to be limited, in any manner, by these recited objects. Rather, the "objects of the invention" are to be used to place the invention in proper overall perspective, to enable the reader to gain an understanding of the various embodiments of the invention and to allow the reader to better understand the manner in which the invention is made and used, especially in its preferred embodiment(s). Accordingly, the various "objects of the invention" are set forth below.

It is a first object of this invention to provide a technique, including but not limited to, at least one method and at least one an apparatus, for eliminating and/or substantially reducing the amount of nitrate containing compounds in a target medium and/or substance and/or environment.

It is a second object of this invention to provide a technique, including but not limited to a method and an apparatus, for eliminating and/or substantially reducing the amount of nitrate containing compounds in a target medium and/or environment, which overcomes many, if not all, of the various previously delineated deficiencies of the prior art.

It is a third object of this invention to provide a technique, including but not limited to at least one method and at least one apparatus, for eliminating and/or substantially reducing the amount of nitrate containing compounds in a target medium and/or environment, which overcomes many, if not all, of the various previously delineated deficiencies of the prior art.

It is a fourth object of the invention to provide a technique, including but not limited to at least one method and at least one apparatus, for eliminating and/or substantially reducing the amount of nitrate containing compounds in a target medium and/or environment, which overcomes many, if not all, of the previously delineated deficiencies of the prior art and which utilizes nitrate reductase.

It is a fifth object of the invention to provide a technique, including but not limited to at least one method and at least one apparatus, for determining the concentration and/or level of nitrate present in a target medium and/or environment.

It is a sixth object of the invention to provide a technique, including but not limited to at least one method and at least one apparatus, for determining the concentration and/or level of nitrate present in a target medium and which overcomes many, if not all, of the previously delineated deficiencies of the prior art, and which is relatively inexpensive and/or cost effective and which is relatively easy to use.

It is a seventh object of the invention to provide a technique, including but not limited to at least one method and at least one apparatus, for determining the concentration and/or level of nitrate present in a target medium and/or environment, which overcomes many, if not all, of the previously delineated deficiencies of the prior art, which utilizes nitrate reductase, and which may be used with water and/or various foods.

It is an eighth object of the present invention to provide a technique for water treatment and/or purification which may be used as part of or which may comprise a municipal water facility.

According to the teachings of a first aspect of the present invention, a method for extracting, preparing, and storing nitrate reductase is provided. In one embodiment, the method comprises the steps of obtaining a quantity of corn seeds; planting said quantity of corn seeds in a container having a certain pre-selected amount of vermiculite; placing said container upon a flat surface; exposing said container to a high intensity fluorescent light; preparing a solution of Ammonium Nitrate and Molybdate; spraying said solution upon said vermiculite; harvesting the leaves of corn plants produced by said seeds; grinding said harvested corn leaves; mixing the ground material with ZM2,69 monoclonal antibody which has been prepared by loading a certain amount of immunoglobulin G onto a quantity of cyanogen bromide activated Sepharose, thereby creating nitrate reductase; placing said nitrate reductase into a second container having a certain and pre-selected amount of MOPS at a pH of about 7.5; and placing said second container into a refrigerator which maintains the temperature of the second container at about -80 degrees centigrade.

According to the teachings of a second aspect of the present invention, a method for determining the amount and/or concentration of nitrate containing compounds in a target medium is provided. The method, in its preferred embodiment, utilizes the nitrate reductase which is produced in the manner specified in the description of the first aspect of the present invention. In the preferred embodiment of the invention, the method comprises the steps of providing a first group of test tubes; providing a second group of test tubes, each of the first and second group of test tubes being substantially free of nitrate, nitrite, and nitrate containing compounds; placing a substance having a pre-determined amount of nitrate into said first group of test tubes; placing a substance having an unknown amount of nitrate into said second group of test tubes; adding a pre-determined amount of substantially nitrate free de-ionized water to each of said groups of test tubes; adding to and mixing with the substances within each of said group of test tubes a pre-determined amount of buffer, NADH solution, and nitrate reductase ("NaR") solution; allowing the reaction to proceed in each of said groups of test tubes for about fifteen to about twenty minutes at a temperature of about 30 degrees Centigrade; agitating each of said groups of test tubes for a pre-determined amount of time; adding a certain amount of color reagent to each of said groups of test tubes; placing a certain amount of the substance contained in said first group of test tubes into a cuvette and the absorbance at about 540 nm is measured; plotting the absorbance readings received from said first group of test tubes against the concentration of nitrate in each of said first group of test tubes, effective to establish a calibration curve; placing a certain amount of the substance contained in each of said second group of test tubes into a cuvette and measuring the absorbance of each of said substances at about 540 nm; and comparing the absorbance measurements associated with each of said test tubes in said second group against said calibration curve, effective to determine the amount of nitrate in each of said second group of test tubes.

According to the teachings of a third aspect of the present invention, a home test kit is provided which utilizes the technique and/or methodology previously delineated with respect to the first and second aspects of the present invention. In yet a second embodiment of this second aspect, the cuvette is replaced with color means for producing a color to determine the amount of nitrate present in various substances.

According to the teachings of a fourth aspect of the present invention, a water treatment system is described. The system includes a generally hollow and columnar container into which are removably positioned and cascaded filters which communicate with water and cooperatively remove nitrate therefrom.

Further objects, features, and advantages of the present invention will become apparent from a consideration of the following description, the appended claims, and/or the appended drawings. It should further be realized by one of ordinary skill in the art that the previous delineated objects and aspects of the invention are for illustration purposes and are not to be construed so as to limit the generality of the inventions or to limit the interpretation to be given to the various claims.

### BRIEF DESCRIPTION OF THE DRAWINGS

Various advantages of the present invention will become apparent to those of ordinary skill in the art by reading the following description and by reference to the following drawings in which:
Figure 1 is a schematic diagram of a home test kit made in accordance with the preferred embodiment of the invention;
Figure 2 is a schematic diagram of a color chart used in combination with the home test kit which is schematically shown in Figure 1;
Figure 3 is a graph illustrating the activity level of the nitrate reductase enzyme which has been freeze dried and used in accordance with the principles of the preferred embodiment of the invention;
Figure 4 is schematic diagram of a water purification system which is made in accordance with the teachings of the preferred embodiment of the invention; and
Figure 5 is a plan unassembled view of one of the water purification filters which forms a portion of the invention shown in Figure 4; and
Figure 6 is a top plan view of the filters shown in Figure 5; and
Figure 7 is a plan view of a nitrate reductase containing bag made in accordance with the teachings of the preferred embodiment of the invention.

### DETAILED DESCRIPTION OF THE INVENTION

### (a) Nitrate Reductase Production and Storage

In order to achieve the various objects and aspects of the invention, Applicants have found it to be first necessary to produce and/or extract and/or purify nitrate reductase which, as will be known to those of ordinary skill in the art, is a naturally occurring enzyme which is found within plants, fungi, and algae. The foregoing method of nitrate reductase production, as Applicants have found, produces relatively highly pure and stable nitrate reductase which may be used to measure nitrate containing compounds.

In the preferred embodiment of the invention this nitrate reductase is produced by initially obtaining corn seeds produced and/or sold by The Downing Foundation Seed Company and having a type designation of DM2×DF20. These seedlings are placed in commercially available flat type containers and planted in commercially available medium grade type vermiculite. In one embodiment of the invention, each flat is comprised of plastic and has dimensions of about 11"×20"×5", has about two-thirds, by volume, of vermiculite and contains about two hundred milliliters of corn seeds per flat over which this vermiculite is placed. Each flat is then watered with conventional tap water until water is seen slowly dripping through each of the flats.

Each of the flats is placed in a flat and stationary position for about three days, at a temperature of about eighty degrees centigrade, in low light conditions. On about the fourth day, each of the seed containing flats is placed under a six foot fluorescent light of the ultra-high or very high output type for about fifteen to about twenty-four hours. On each of the first four days, each flat is watered, with conventional tap water, to ensure that the vermiculite remains moist. On the fifth and sixth day, each of the flats are sprayed with a solution prepared in accordance with the teachings of the preferred embodiment of the invention. That is, in the preferred embodiment of the invention, the solution is made or mixed within a sprayer having a volume of about 26 liters. Into this sprayer is placed about 51/2 tablespoons of commercially available Miracle-Gro™ and about 550 milliliters of conventional and commercially available 1 Molar Ammonium Nitrate Solution. The sprayer is then filled with conventional tap water and is used to spray about 12 flats. In one embodiment of the invention, the spraying is done once between 3:00 P.M. and 7:00 P.M. each of the fifth and sixth days. Moreover, during each of these fifth and sixth days, the seedling containing flats are each placed under the previously delineated fluorescent lights from about 8:00A.M. to about 10:00P.M. On the seventh day, between about 9:00A.M. and about 11:00 A.M., the leaves of each of the grown corn plants are sheared and harvested with commercially available electric shears. The harvested leaves are stored at a temperature of about -80 degrees centigrade until the harvested leaves are used to produce the nitrate reductase substance of the invention.

In order to prepare the relatively highly stable and highly pure nitrate reductase enzyme, according to the teachings of this invention, about five to about six kilograms of frozen corn leaves are placed into a commercially available Waring type blender, having a volume of about 4 liters. About six to about seven liters of 0.1 M potassium phosphate, having a pH of about 7.5, containing about 1 mM commercially available EDTA and about 5 mM cysteine-HCI is also placed into the Waring blender. Further, in the preferred embodiment of the invention, about 25 g of commercially available polyvinylploypyrrolidone is added to the blender for each kilogram of frozen corn leaves. The mixture is blended, in the preferred embodiment of the invention, for about sixty seconds at the setting of "high". The ground mixture is then filtered through about two to about four layers of commercially available grade 70 cheesecloth which is obtained from the Fisher Scientific Company of Pittsburgh, Pennsylvania and about one to about two layers of Miracloth™ which is obtained from the Cal Biochem Company of Menlo Park, California.

In the preferred embodiment of the invention, the resulting filtrate is passed to a commercially available flow through rotor manufactured by the Beckman Instruments Company and having a model number of JCF-Z. The rotor further includes a Beckman model JZZ1MI centrifuge which operates at a speed of about 10,000 RPM. The centrifuged extract is mechanically stirred at a temperature of about 4 degrees centigrade with about 30 ml of modified monoclonal antibody Zm2,69-Sepharose per liter of extract.

That is, Applicants have found that the modified preparation of the Zm2,69 monoclonal antibody allows for a substantially purer and more stable nitrate reductase production than was previously accomplished and/or is known. Specifically, the Zm2,69 monoclonal antibody Sepharose is prepared in substantially the same manner as described in the article entitled Monoclonal antibody-based immunoaffinity chromatography for purifying corn and squash NADH: nitrate reductase. Evidence for an interchain disulfide bond in nitrate reductase, which was published on pages 233-246 of Plant Molecular Biology, by Kluwer Academic Publishers, in 1989, which is authored by Gregory Hyde, Julie Wilberding, Annette Meyer, Ellen Campbell, and Wilbur Campbell (Applicant of this Patent Application) and which is fully and completely incorporated herein by reference, word for word and paragraph for paragraph, except that the antibody is loaded onto the cyanogen bromide activated Sepharose at about 0.5 mg immunoglobulin G per milliliter of gel. This modification in the preparation of the Zm2,69 monoclonal antibody gel is regarded by Applicants as novel, unobvious over the prior teachings, and in fact, allows for the objects of the invention to be achieved. After about 15 to about 60 minutes of mixing, the monoclonal antibody Zm2,69-Sepharose is recovered by means of vacuum filtration and repeatedly washed with about 0.1 M of commercially available potassium phosphate, having a pH of about 7.5 and containing about 1 mM of commercially available EDTA. After the monoclonal antibody Zm2,69-Sepharose has been washed with a total of about 5 liters of buffer, it is suspended in the same buffer and packed into a glass column and allowed to settle with buffer effluent flowing. In one embodiment of the invention, the glass column has a height of about 30 cm. and a width of about 2.5 cm. When the monoclonal antibody Zm2,69-Sepharose is settled and the buffer reservoir depleted, the nitrate reductase is eluted by application of about 0.01 M of commercially available glycine, having a pH of about 11. Five milliliter fractions are collected in tubes which are previously loaded with about 0.05 ml of about 1M of commercially available MOPS, having a pH of about 7. As should be apparent to one of ordinary skill in the art, the term "MOPS" denotes 3[N-morpholino]propanesulfonic acid which is adjusted to a pH of 7 by application of commercially available NaOH. The packing of the monoclonal antibody gel with bound enzyme into the glass column and its elution with glycine is accomplished at a temperature of about 4 degrees centigrade. The fractions are then mixed by inversion in order to achieve homogeneity, the activity for reducing nitrate to nitrite in the presence of commercially available NADH and the absorbance of each fraction at 413 nanometers is measured. The fractions are stored at about negative 80 degrees centigrade. Subsequently, the fractions are thawed, pooled, and concentrated using a commercially available centrifugal concentrating device or commercially available pressure concentrating device, each of which allow the buffer to be exchanged in order to remove the residues of phosphate, glycine, and various other types of chemicals present during the previously delineated purification process. In the preferred embodiment of the invention, the final preparation of nitrate reductase is placed within about 50mM MOPS, pH 7.5 (Na form). The final concentration of the prepared nitrate reductase is about ten units of nitrate reductase activity per milliliter with the unit activity defined as the ability to convert about one micromole of nitrate to nitrite per minute. The concentrating and buffer exchange process, forming an integral part of the preferred embodiment of the invention, is found to restore activity of the enzymes lost during processing such that greater quantities of enzyme activity are found after the buffer exchange and concentrating activities are completed, then directly after the completion of the purification procedure described earlier in the application.

Applicants have found that the environment in which the prepared nitrate reductase is stored and placed is crucial to the prepared nitrate reductase's ability to reduce, eliminate, and/or determine the concentration of nitrates in a target solution. In the preferred embodiment of this invention, the produced/prepared nitrate reductase is placed within a refrigerator which maintains a relatively constant temperature of about negative 80 degrees centigrade and is further placed within about 50 mM MOPS having a pH of about 7.5. Applicants have further found that the prepared and produced nitrate reductase may be stored at a far higher temperature of about -20 degrees centigrade provided that an substantially equal volume of 100% glycerol (molecular biology grade) is added to a substantially equal volume of nitrate reductase solution. Applicants have also discovered that the produced and prepared nitrate reductase may also be stored at substantially room temperature provided that the nitrate reductase is mixed with molecular biology grade sucrose, which is also substantially free of RNAse. In the preferred embodiment of the invention, about 40 grams of the sucrose is mixed with about 40 ml of the prepared and produced nitrate reductase. Particularly, the nitrate reductase-sucrose solution is freeze-dried, by known and conventional techniques, to a glass state.

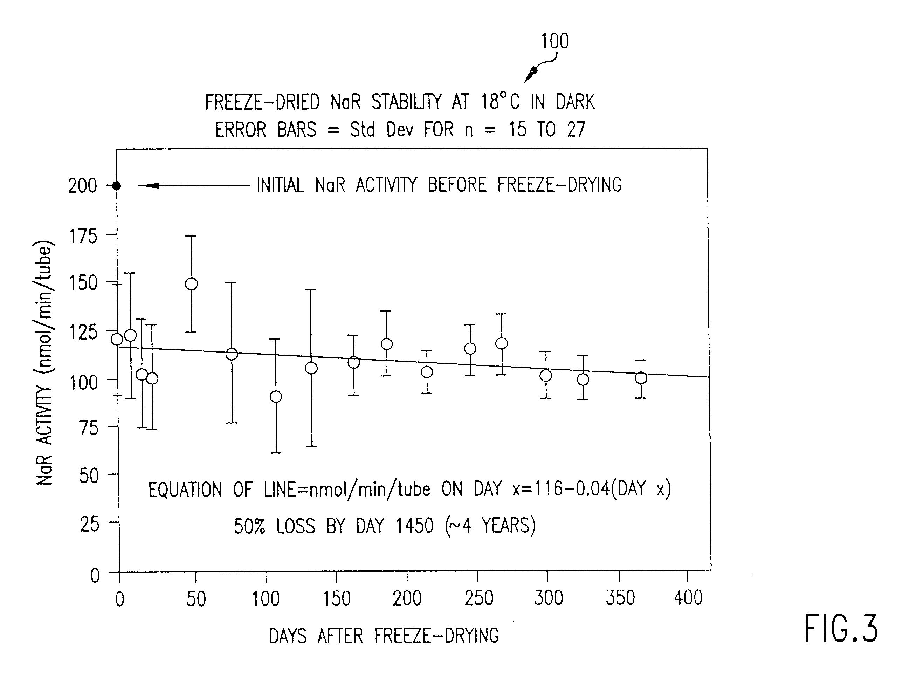

Referring now to Figure 3, there is shown a graph 100 which represents data experimentally developed by Applicants and associated with the experimentaliy measured and experienced stability of the freeze dried nitrate reductase. As shown graph 100 displays the experimentally derived/measured activity of the freeze dried nitrate reductase over a time period measured or quantified by a metric known as "days after freeze drying". As shown in Figure 3, and more particularly in graph 100, the initial non-freeze dried nitrate reductase has an activity level of about 200 nmol/min/tube and that the freeze dried nitrate reductase has an activity level in the range of about 175 to about 60 nmol/min/tube over an about 400 day period, which is very acceptable. The experimental results shown in graph 100 support Applicants' use of the freeze dried nitrate reductase in the manner contemplated and explained in this Application.

Applicants have found that such "freeze drying" allows the nitrate reductase to be easily stored and/or placed within Applicants' various embodiments and that such storage and placement do not substantially and adversely affect the overall system operation. Hence, the "freeze drying" of the nitrate reductase produces the unexpected result of allowing for a relatively large amount of chemical activity (non-performance degradation) while providing for a relatively easy to use system.

### (b) Nitrate Determination Techniques (Non-Home Based)

Applicants have found that nitrate reductase produced and prepared in accordance with the afore-described techniques of the invention may be employed in several techniques and/or methodologies and apparatuses which are effective to allow one to determine the nitrate concentration or level in various target substances, such as water and which may be further used in a large scale water treatment system.

The various non-home based assay techniques "work" or perform their function by "turning" nitrate into nitrite with an enzyme (e.g. nitrate reductase) and then measuring the produced nitrite by use of various reagents, such as and without limitation, the known and commercially available Griess type reagents.

The first such nitrate determination technique comprises a colormetric concentration determination method and apparatus. In this first technique, two groups of test tubes are initially employed. In the preferred embodiment of the invention, each of the employed test tubes are comprised of glass and/or borosilicate type compounds and are substantially nitrate and nitrite free. Moreover, in one embodiment of the invention, each of the test tubes has a diameter of about 13 mm and a height or length of about 100 mm. A water sample containing an unknown amount of nitrate is added to each of the test tubes in the first group. About 0.05 µl of commercially available and substantially conventional nitrate standard was prepared in the range of about 0 to about 15 ppm of Nitrate-N ml is added to each of the test tubes in the second test tube group. As should be realized by one of ordinary skill in the art, the "standard" nitrate solution contains, in one embodiment of the invention, about 15 ppm of nitrate.

To each of the test tubes of each of the test tube groups, about 0.45 ml of conventional and commercially available and substantially nitrate free de-ionized water is added and mixed. After the de-ionized water is added and mixed with the initial test tube contents, about 0.3 ml of buffer solution is added to each of the test tubes and mixed, then about 0.1 ml of NADH solution is added to each of the test tubes and mixed with the test tube contents, and then about 0.1 ml of NaR solution is added to each of the test tubes and mixed with the test tube contents. In the preferred embodiment of the invention, each of the foregoing substances/materials are sequentially added and mixed with the contents of each of the test tubes, however, alternatively, each of the foregoing substances/materials may be added and mixed in a substantially simultaneous manner and/or in a different order from that set forth above. In fact, Applicant has found that the order in which these last three substances are added to the test tubes is of no consequence. In the preferred embodiment of the invention, after all of the afore-described addition and mixing is completed, each of the test tubes has a substantially equal total volume of about 1.0 ml.

In the preferred embodiment of the invention, the afore-described buffer solution comprises about 0.1 M potassium phosphate which is itself prepared from grade KH2PO4 (ACS reagent agent grade) with pH adjusted with KOH (ACS reagent grade), in substantially nitrate free de-ionized water. In the preferred embodiment of the invention, the buffer solution may also contain about 0.00005 M of conventional and commercially available EDTA which, as should be apparent to one of ordinary skill in the art, is a chelating reagent which is used to protect the nitrate reductase against inhibition by the various known heavy metal contaminants of the various salts which are contained in the buffer solution. In the preferred embodiment of the invention, the NADH solution comprises 2 mM NADH (beta-nicotinamide adenine dinucleotide, reduced form) which may be obtained from the Sigma Chemical Company and is referred to as product number N8129 prepared in substantially nitrate free de-ionized water.

After the solutions are added to each of the test tubes and completely mixed, the reactions are allowed to proceed for about 15 to about 20 minutes at substantially room temperature. Alternatively, the reactions may proceed at a temperature of about 30 degrees centigrade. However if the reactions proceed at a temperature of about 30 degrees centigrade, Applicants have found it to be advantageous to "pre-heat" or "pre-warm the various test tubes prior to the addition of the nitrate reductase. At the termination of the reaction, about 0.5 ml of a color reagent is added to each of the test tubes and thoroughly mixed with the contents of each of the test tubes. In the preferred embodiment of the invention this color reagent is produced by mixing about 10 g of sulfanilamide, commercially available from the Sigma Chemical Company and denoted as Product number S9251 (ACS reagent grade) with one liter of 3 N HCI (ACS reagent grade) and with substantially nitrate free de-ionized water. Alternatively, about 1 M of citric acid, commercially available from the Sigma Chemical Company and denoted as product number C7129, may be utilized in place of the 3 N HCI. After this mixing is completed, a second color reagent is added to each of the test tubes. In the preferred embodiment of the invention, this second color reagent produced by mixing about 200 mg N-naphthylethylenediamine-dihydrochloride, commercially available from the Sigma Chemical Company and denoted as product number N9125 with one liter of substantially nitrate free de-ionized water. This second reagent is mixed with the substances contained in each of the various test tubes. In the preferred embodiment of the invention, each of the test tubes, at this stage of the process, contains about 2.0 ml of solution.

After the test tubes have been allowed to stand for about 15 to about 20 minutes, they are each agitated in order that their respective contained substances are completely mixed. Amounts of solutions from each of the test tubes are transferred to a commercially available cuvette container and the absorbance at a wavelength of about 540 nanometers is observed using, in one embodiment of the invention, a commercially available colorimeter or spectrophotometer. The absorbance readings from the first group of test tubes are plotted with respect to the amount of nitrate (in ppm) contained in each of the first group of test tubes and a calibration curve is generated. Using the generated calibration curve and the measured absorbances associated with each of the test tubes from the second test tube group, one interpolates the calibration curve to determine the amount of nitrate present in each of the second group of test tubes.

Applicants have found that one of the major advantages of this technique, in addition to producing highly accurate nitrate determination results, is that all of the afore-described reaction mixtures and analyzed samples may be safely disposed of by means of a public sewer system since each of the afore-described solutions are relatively safe to the environment as long as they are properly diluted with a large volume of tap water prior to disposal. Moreover, Applicants have further found that this afore-described method "cancels" or "negates" any nitrate contribution added to the test tubes by any of the reagents or enzyme, thereby making the process more accurate and simple.

In a second embodiment of the nitrate determination aspect of the present invention substantially similar dual groups of test tubes as earlier described are utilized. As before, the first group of these test tubes contains a water sample having a known amount of nitrate. The second group contains a water sample of an unknown amount of nitrate. Substantially nitrate-free de-ionized water is again added to each of the test tubes and all the samples are thoroughly mixed and/or agitated.

In this second embodiment, several reagents and substances are sequentially added to each of the test tubes and thoroughly mixed after each addition. The first such substance/reagent is 0.3 ml of substantially the same buffer solution as employed in the first process embodiment of the invention. The second substance/reagent comprises about 0.05 ml of a methyl viologen solution which, in the preferred embodiment of the invention, is created by combining about 31 mg of methyl viologen, commercially available from the Sigma Chemical Company and denoted as product number M2251, with one milliliter of substantially nitrate free de-ionized water. As should be apparent to one of ordinary skill in the art, other known electron carrying dyes may be used in place of the methyl viologen solution. The third substance/reagent is substantially the same nitrate reductase solution which was used in the first embodiment. Lastly, the last such substance is about .05 ml of dithionite solution which is produced by combining about 35 mg of sodium dithionite (sodium hydrosulfite which is commercially available from the Sigma Chemical Company and denoted as product number S1256) with about one milliliter of 0.1 N NaOH which is prepared from substantially nitrate free de-ionized water.

The test tube solutions turn a deep color of blue after the dithionite is added and remains this color of blue during the reaction incubation time. Particularly, Applicants have noted that the incubation time is about 15 to about 20 minutes and that the reaction, as in the first embodiment, should proceed at either room temperature or at a temperature of about 30 degrees centigrade. After this time has expired, each test tube is vigorously shaken and/or mixed until the deep blue color is substantially gone. After the deep blue color is substantially gone, the remaining steps associated with the various previously described color reagents, colorimeter/spectrophotometer, and calibration curves are substantially similar to those of the first process embodiment of the invention.

Applicants have discovered that each of the previous two nitrate determination embodiments provides relatively highly accurate concentration results when the nitrate level of the "unknown" water samples (comprising the second group of test tubes) is within the range the nitrate levels in the first group of test tubes- the test tube samples used to produce the calibration curve. If nitrate levels are higher than the levels of the standardized test tubes, the calibration curve technique may yield an underestimation of concentration values unless the samples are diluted Hence, with high nitrate containing water samples (e.g. although the nitrate concentration of these samples is unknown, it is determined to be above about 10 ppm), Applicants have found it to be both beneficial and useful to add about 9.0 ml of substantially nitrate free de-ionized water to each milliliter of sample. The nitrate concentration determined by means of the previously described calibration curve is then multiplied by a factor of ten to yield the correct nitrate concentration value.

In yet a third nitrate determination embodiment of the invention, conventional and commercially available 96-well microtiter plates are used. In this technique and utilizing the provided nitrate reductase, which is purified and prepared in the previously explained manner, a relatively highly accurate nitrate concentration determination is provided. All of the obtained microtiter plates have wells which are substantially free of nitrate, nitrite, and compounds and/or solutions containing nitrate and/or nitrite. From one to about six wells are assigned to contain each water sample to be analyzed. Into each of these first assigned wells is deposited about 0.01 milliliters of a water sample containing an unknown amount of nitrate. From one to about six other wells are assigned to contain a solution containing a known amount of nitrate. These wells serve as the nitrate "standards", thereby forming a second set of wells. Into each well of this second set of wells is deposited about 0.01 milliliter of the nitrate standard.

A third set of wells or microtiter plates is formed by allocating from about one to about six other wells or microtiter plates to serve as background control wells. Specifically, into each of these wells forming this third well set is deposited about 0.01 milliliters of substantially nitrate free water.

To each well in each of all three well sets is added about 0.07 milliliters of buffer solution (50 mM MOPS, 1 mM EDTA at pH 7.0), about 0.01 milliliters of NADH solution (8 mg NADH in 5 milliliters of deionized water), and about 0.01 milliliters of a nitrate reductase solution which contains about 2 Units per milliliter. The nitrate reductase was prepared in the previously delineated manner. The afore-described solutions may be added sequentially to each of the wells or in a substantially simultaneous manner. Applicants have found that the order in which these solutions are placed into each of these wells is of no consequence for purposes of Applicants' invention. After these solutions are placed into each of the wells of each of the three sets, mixing occurs by use of a conventional and commercially available microtiter plate shaker apparatus. Mixing and incubation is done at ambient temperature, between about 20 degrees Centigrade to about 30 degrees Centigrade, for a period of a minimum of 15 minutes.

After the mixing step, each of the wells or microtiter plates are subjected to substantially the same reagents as was the test tubes in the first and second embodiments of the invention. That is, 50 microliters of commercially available sulfanilamide reagent (10 grams sulfanilamide dissolved in 1 liter of 3 N HCI) and 50 microliters of N-(1-naphthyl)ethylenediamine dihydrochloride reagent (200 milligrams dissolved in 1 liter of deionized water) are added to each well. The microtiter plate is again mixed with the assistance of the microtiter plate shaker apparatus for a period of at least 10 minutes, at ambient temperature. The resulting color is read using a commercially available microtiter plate reader at an absorbance wavelength of 540 nm.

It should be apparent to those of ordinary skill in the art that the foregoing microtiter plate assay embodiments may be independently used wholly apart from any other aspect or embodiment of this invention.

### (c) Home Based Nitrate Determination Techniques

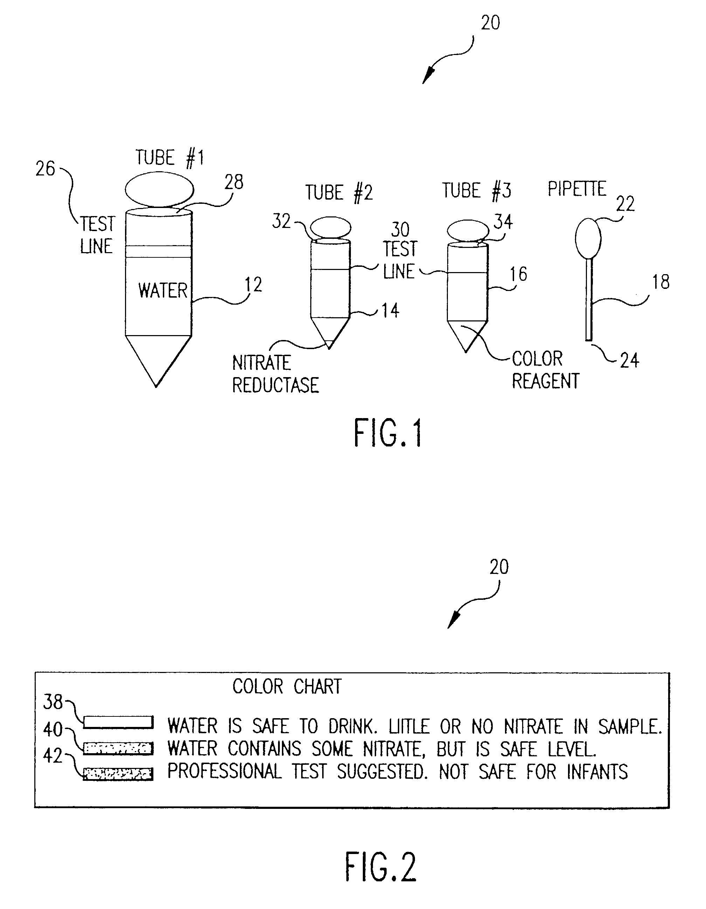

Referring now to Figure 1, there is shown a Nitrate determination and/or concentration determination home based testing kit made in accordance with the teachings of the preferred embodiment of the invention. As shown, test kit 10 includes three test tubes 12, 14, and 16 and two conventional and commercially available pipettes 18. In its most preferred embodiment, Applicants' home-based testing kit 10 includes these components in addition to the color chart 20 which is schematically shown in Figure 2, an instruction sheet (not shown) but describing the operation of the home-based testing kit in accordance with this description. All of these kit elements and/or components are preferably contained and/or housed within a single box.

Particularly, in the preferred embodiment of the invention, test tube 12 contains about 9 milliliters of substantially nitrate free water and, is made of substantially clear plastic, and has a volume of about 15 milliliters. Test tube 14 contains nitrate reductase which most preferably has been prepared in accordance with the principles and methodologies previously delineated in this application. In the most preferred home-based testing apparatus of the invention, the produced/synthesized nitrate reductase is freeze-dried within test tube 14. Also included in test tube 14 is sufficient dry NADH reagent to drive the enzymatic reaction. Test tube 14, in the most preferred embodiment of the invention, is a 1.5 milliliter standard polyethylene or polypropylene microfuge tube. Moreover, test tube 16 contains about 75 microliters of the color reagent solution, which is comprised of about 10 grams of sulfanilamide and 200 milligrams of N-(1-naphthyl)ethylenediamine dihydrochloride dissolved in one liter of 3 N HCI.

In operation, the tip 24 of one of the clean pipettes 18 is placed within the water sample to be tested. The bulb 22 is squeezed and released to substantially fill pipette 18 with sample water. Part of the obtained water sample is squirted or placed within test tube 12 sufficient to full test tube 12 up to test line 26. Cap 28 is placed upon test tube 12 and test tube 12 is shaken and with a second pipette 18, enough liquid is extracted from tube 12 to fill tube 14 to the test line 30. Cap 32 is placed upon tube 14 and the tube is shaken occasionally for about ten minutes. The shaken contents of tube 14 are transferred to tube 16. Cap 34 is placed upon tube 16 and tube 16 is shaken. After tube 16 is stationary for about five minutes, tube 16 is quickly shaken again and the color inside recently shaken tube 16 is matched with color chart 20.

As shown, chart 20 has three rectangular color entries 38, 40 and 42 respectively indicating that the water is relatively safe to drink since there is little or no nitrate containing compounds within the sample; the sample water contains some nitrate containing compounds but is still considered relatively safe; and the water may not be safe and the homeowner should obtain a more detailed test. As should be apparent to those skilled in the art, color chart 20 may be readily replaced with three nitrate color standard tubes containing different amounts of representative colors with labels indicating the amount of nitrate present in the water sample with a comparable color. It should be apparent that the foregoing home-based nitrate determination test is relatively easy to use, inexpensive, and of great utility.

A similar procedure may be employed to obtain the nitrate concentration levels within food. The food must be initially be blended in nitrate-free water either provided in the kit or obtained by user. The extract obtained from the blender must be filtered using a commercially available cheesecloth provided in the kit or provided by the user. Liquid food extracts, such as carrot juice, need not be filtered.

Secondly, a tube is filled with a predefined amount of the food extract after filtration and the tube is capped. The tube is then placed in boiling water for about five minutes. If the tube has lost volume during boiling, the lost liquid must be replaced with nitrate-free water. If excessive particles form, it may be necessary to filter the boiled food extract simple again.

Thirdly, the boiled food extract sample will now be treated as a water sample and the procedure described above from home based water testing and, in the foregoing manner, the kit will be used to estimate the nitrate content of the food extract.

Specifically, the food is prepared by chopping or dicing and blend it with an appropriate amount of nitrate-free water. Estimate the volume of food extract in ml using a measuring cup. The food extract is transferred to the Fill Line in the Boiling Tube. The tube is capped and placed in boiling water for five minutes.

The boiled samples are allowed to cool. If the samples contain only small amounts of particles, then transfer the liquid contents to the Sample Dilution Tube (after any water that evaporated during boiling and cooling is replaced with nitrate-free water) to Test Line using one of the plastic pipettes. After the sample in the Sample Dilution Tube is mixed and any particles are allowed to settle, then transfer an amount sufficient to fill the Reaction Tube to the Fill Line and mix thoroughly by inverting the tube about three times. Allow tube to stand for twenty minutes at room temperature (20 to 30 degrees Centigrade).

Transfer the liquid contents of the Reaction Tube to the Color Development Tube and mix completely. Allow about fifteen minute for color to development. Compare the color in the Color Development Tube on the Color Chart to estimate the nitrate content of the food extract. Or use the Color Standard Tubes to estimate the more exact nitrate content in terms of ppm nitrate-N. The result obtained is for only a fraction of the total food extract prepared or the food extract (i.e. carrot juice). Estimate from the total volume of food extract, the total amount of nitrate in the food source or total food extract volume itself by multiplying the total number of ml of the food extract times the amount of nitrate estimated to be in the test sample, which is considered to have a volume of 1 ml. This total amount of nitrate-N can be translated into "water equivalents" to gain an idea of how much of the nitrate contaminated food or food extract is safe to consume. There are no set national standards for food and food extra nitrate content limits. If a carrot was blended and its extract found to have a total volume of 100 ml, then the nitrate content of the sample analyzed would be multiplied by 100 to obtain the total amount of nitrate in the carrot. So if the 1 ml of carrot extract was found to have 5 ppm nitrate-N, then eating the carrot would be equivalent to drinking 100 ml of water with 5 ppm nitrate-N. The user can then take this information into consideration to evaluate the safety of eating carrots with this nitrate content. It should be apparent that what has been described as a relatively easy to use "home based" nitrate determination system which may be used in a manner wholly independent from any other embodiment of the invention.

### (d) Water Purification System

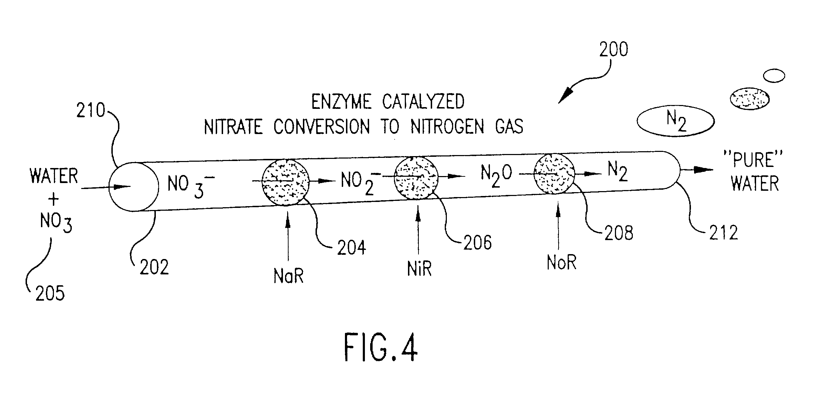

Applicants have found that the nitrate reductase which is made/synthesized and/or purified in accordance with the various previously delineated principles of the invention (which are more fully described earlier in this Application) is useful as part of a water purification and/or water treatment system, and which may be amenable and/or adapted for use by municipalities and other relatively large geographic and relatively largely populated areas. The further principles underlining these water purification embodiments are, for example and without limitation, schematically shown and described with respect to Figures 4, 5, 6 and 7 of this Application.

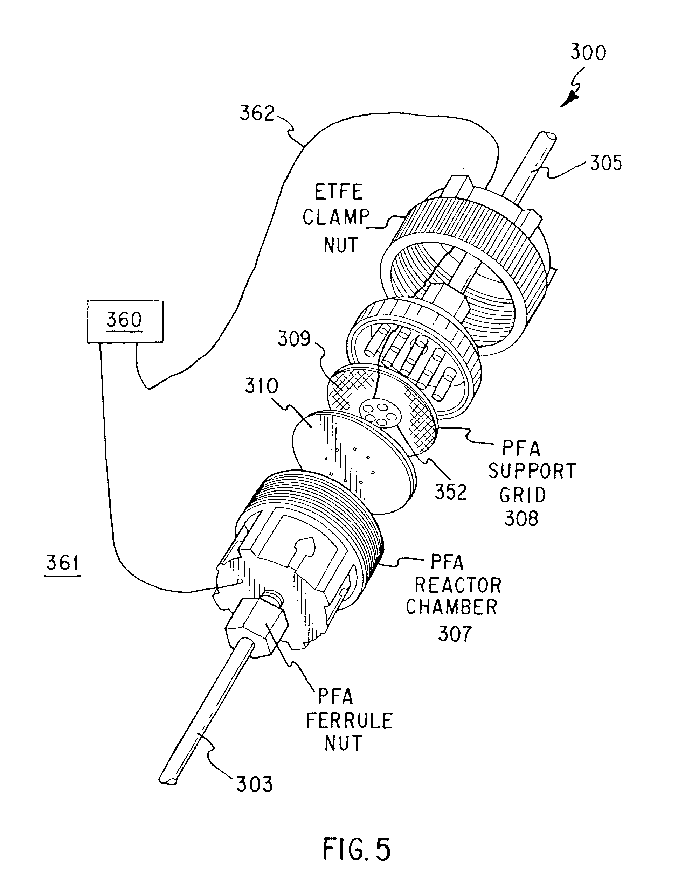

Referring now to Figure 4, there is shown schematically and in a "block diagram" type manner, a water purification apparatus 200 which is made in accordance with the principles of the water purification aspects of the present invention. As shown, apparatus 200, in one embodiment, comprises a generally rounded, hollow, and openended columnar structure or apparatus 202 which is adapted to removably, and longitudinally contain a plurality of treatment filters 204, 206, and 208 which are sequentially, spatially, and/or cascadingly placed within the longitudinal portion of the body of column 202 in the order shown. It should be understood that the order and number of filters 204-208 shown in Figure 4 may be varied in other embodiments of the invention and that this Application should not be construed, and is not meant to be construed, as limiting apparatus 200 to include only the exact number or types of filters and their corresponding placement shown in Figure 4 or to be constrained by the columnar shape shown in Figure 5. Moreover, it should further be realized that the spatial and cascaded distribution of the filters 204-208 within apparatus 200 allows each filter to provide a chemical reaction to material (e.g. water) which has previously been chemically "treated" by reaction by the preceding filters (e.g. filter 208 provides reaction to water which has previously been "reacted" with filters 204 and 206.) In this manner, filters 204-208 cooperatively treat water 205. Thus, Applicants' invention, in one aspect, is directed to a cascaded arrangement of filters or individual "reactors" which provide unique chemically biological reaction to water as it flows through or in close proximity to the filters.

Apparatus 200 is adapted to substantially communicate with and receive "unpurified" or nitrate contaminated water at one open end 210, to channel or communicate the unpurified water through each of the filters 204, 206, and 208 and to output at its distal end 212 substantially "nitrate free" or substantially purified and "treated" water. As should be realized by one of ordinary skill in the art, removable filters 204, 206, and 208 chemically and/or biologically interact with the contaminated water, thereby producing, in a cooperative fashion (nitrate free) substantially pure water. Moreover, the columnar, or "flow through" arrangement allows a relatively large amount of water (or semi-solid waste) to flow through the filters thereby making apparatus 200 very useful in applications requiring large amounts of water or waste to be purified in a relatively efficient manner. In fact, apparatus 200 itself may form part of a municipal water distribution system and be interjected within the physical distribution "pipes" or conduits. The removable nature of the filter placement further allows apparatus 200 to be repeatedly used for water purification even after the initial filters lose their ability to purify the water filters 204-208 are simply replaced as used. The following discussion centers upon the use and the function of the filters 204-208 and upon the use and function of the water purification apparatus 200.

Specifically, in the embodiment of the water purification apparatus shown in Figure 4, three enzymes are required for the stepwise or sequential reduction of the nitrate in the contaminated water to nitrogen gas. In the embodiment shown in Figure 4, nitrate reductase (NaR), in cooperation with an electron carrying dye, and electrical energy, converts the nitrate within the contaminated and untreated water 205 to nitrite. This reaction occurs by means of filter 204. The initially treated water flows past filter 206. Specifically, filter 206 having or containing nitrite reductase (NiR) converts the nitrite to nitrous oxide or another form of nitrogen oxide being eventually (either by the nitrite reductase or an electro-chemical process) converted to nitrous oxide, which is communicated to filter 206. Filter 208, having or including nitrous oxide reductase (NoR), converts the nitrous oxide to nitrogen gas, a relatively harmless product and relatively "pure" (relatively "nitrate-free") water exits from end 212.

Thus, by spatially separating and cascading filters 204, 206, and 208 within apparatus 200, a sequence of discrete chemical reactions occur which cooperatively purify the untreated water. Each of the filters 204, 206, and 208 are connected to a source of electrical energy. Since the energy needed for the aforementioned chemical reactions to occur is provided to the enzymes by electrical current, there is no need to add anything to the water to assist in the elimination of the nitrate ion as a substantially non-toxic gas. Thus, the water purification apparatus which is schematically shown in Figure 4 uses nitrogen metabolism enzymes which are immobilized in substantially columnar flow-through reactor, powered by either a direct electrical current or an alternating electrical current, to substantially eliminate the nitrate pollution and output relatively environmentally safe dinitrogen gas. As should be apparent to one of ordinary skill in the art, Applicants' design allows the water with the contaminating nitrate to flow through the anode (positive pole) of each filter electrode toward the cathode (negative pole) of each filter electrode where the reservoir of immobilized enzyme/electron-carrying dye intercepts the nitrate and catalyzes the reaction. A discussion of the structure of each of the filters 204-208 follows.

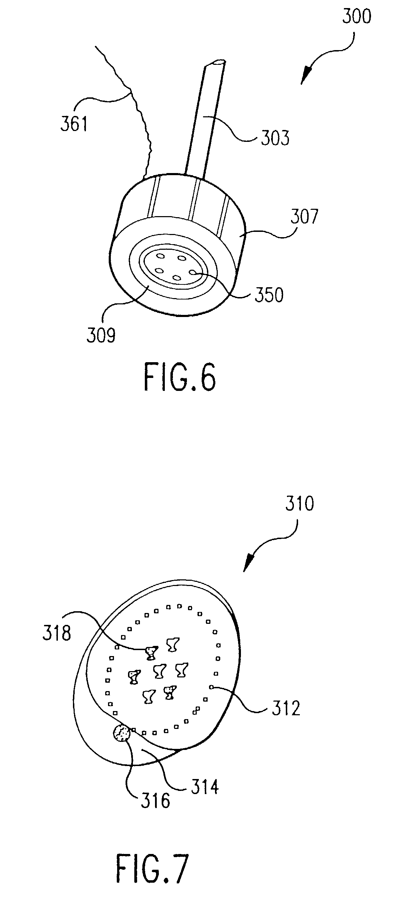

Referring now to Figures 5 and 6, there is shown one example of a filter 300 made in accordance with the teachings of the preferred embodiment of the invention. The structure and operation of filter 300 corresponds to the structure and operation of each of the filters 204, 206, and 208 which were previously discussed.

As shown, exemplary filter 300, in one embodiment, includes or comprises a 50 mm Teflon PFA in-line filter which is commercially available from the Cole-Parmer Instrument Company which is located in Niles, Illinois. As shown, filter 300 includes oppositely extending and removable extensions 303 and 305 which removably attach filter 300 within apparatus 200, two substantially stainless steel electrodes 350 and 352, serving as an anode 350 and a cathode 352 and comprising in one embodiment, an electrode made from stainless steel with about two micron frits which is commercially available from the Alltech Associates Company of Deerfield, Illinois and which has an outside diameter of about one inch and a thickness of about 0.0625 inches. The initial surface area of the electrodes 350 and 352 was about 5.07 cm². Electrodes 350 and 352 are connected to an external electrical power source 360 by electric leads 361 and 362.

In the preferred embodiment of the invention, the stainless steel was obtained in commercially available disk form and the disk was modified by drilling about six holes, each of substantially similar forms and shapes and each having a diameter of about 0.12 inches and by soldering a commercially available platinum wire to the disk as an electrical lead. Thus the final area of the electrode surface was about 4.6 cm². One stainless steel disk with aforementioned holes drilled in it, comprising the positive electrode 350 (anode), was mounted in the end of the reactor chamber 307 in the Teflon filter 300 using commercially available Clear Silicone Sealant 309 which was obtained from the Dow Corning Corporation of Midland, Michigan, in a manner sealing the electrode 350 over the inlet of reactor chamber 307 forcing the contaminated water to pass through the electrode 350, as shown in Figure 6. The negative electrode 352 (cathode) was mounted on PFA support grid 308 in the Teflon filter 300 using commercially available Clear Silicone Sealant 309, in a manner sealing the electrode to the grid 308, and since the stainless steel cathode 352 contained the aforementioned holes drilled in it, the contaminated water was forced to pass through the electrode. Small holes were drilled in the Teflon filter body to allow the electrical leads to exit and the holes were sealed both inside and outside with the Clear Silicone Sealant. After allowing the sealant to cure for about twenty-four hours, the units were assembled with commercially available silicone tubing attached at the inlet and the outlet. The stainless steel cathode 352, which was supported on the support grid 308, of the Teflon filter, was modified with a plastic covered wire to function as a holder for the enzyme bead reservoir 310 to retain it closely over electrode 352 forcing the contaminated water to pass into the enzyme reservoir 310 wherein the enzyme beads are energized by the negative charge of the cathode 352.

In the preferred embodiment of the invention, each enzyme was co-immobilized onto and/or into the filters 204, 206, 208 with commercially available electron carrying dye which in one embodiment comprises commercially available and conventional azure A type dye. Applicants have found that the dye and enzyme may be placed within each of the filters 204, 206, 208 by a number of different solid supports including but not limited to cyanogen bromide-activated Sepharose (CNBr-Seph) which is a compressible gel composed of agarose and cross-linked to enhance stability, which is commercially available from the Pharmacia Fine Chemicals Company of New Jersey. Applicants have found that cyanogen bromide activation makes the gel reactive with amino groups in proteins and other similar types of chemicals. Secondly, Applicants have found that Reacti-Gel (HW-65) also functions relatively well as an immobilizer. Specifically, this gel is relatively non-compressible and is comprised of polyvinyl which is commercially available from the Pierce Corporation of Rockford, Illinois. Applicants have found that the HW-65 gel is activated by carbodiimidazole which will covalently couple amino groups of proteins and other chemicals. Applicants have found that this gel has several advantages over the CNBr-Seph gel in making a non-charged linkage which is relatively leak resistant. However, this gel is relatively expensive and has a lower capacity, than the CNBr-Seph gel, to bind proteins.

Applicants have discovered that perhaps the best immobilizer, to date, is or comprises granular diatomaceous earth activated with polyimine, commonly referred to as "GDE". Specifically, GDE is a relatively non-compressible gel which is composed of diatomaceous earth particles provided, in one instance, by Solvay Enzymes, Inc. of Elkhart, Indiana. While Applicants have found that this gel is less reactive than the previously two specified immobilizers, it has been found to have several advantages in that it may be readily approved for applications in potable water since it is essentially inert and is available in bulk quantities at very reasonable prices, to date.

In operation, the GDE beads are used as a substrate or foundation for the placement of the dye and the particular enzyme that each of the filters 204, 206, and 208 contains. These enzyme and dye carrying beads are placed upon electrode 352 by means of an enzyme reservoir comprised of a cloth bag, such as bag 310, which were sewn with a tight stitch in the general form of circle 312 having a diameter which is substantially equal to the diameter of the electrode end 352, as shown in Figure 7. A small gap 316 was left in the stitching to allow the GDE beads 318 to be put in bag 310 using, in one methodology, a pipette tip as a funnel. Since the cloth of the enzyme reservoir had "flaps" 314, which extended beyond the stitching 312, the filling gap 316 was substantially self-sealing and no additional stitching was required to close the gap after filling the reservoir with GDE beads. Applicants have found that the bag 310 supported the beads upon the electrode yet allowed the incoming contaminated water to contact the beads in order that contained enzymes could appropriately and chemically react with the contaminated water. Applicants have experimentally verified the operation of system 200 and these results are discussed below.

### (e). WATER TREATMENT EXPERIMENTAL VERIFICATION

### (1) Reactor Preparation

The first filter or "reactor" was "setup" with 2 ml NaR/azure-A GDE in a cloth reservoir. The NaR/azure-A GDE sample was taken from the second batch made on January 8, 1995, with about 8 units NaR activity/ml matrix (1 unit = 1 µmole of nitrite produced per minute). This NaR/azure-A GDE preparation was shown to have dithionite-only:NaR activity of 2.8 units/ml of matrix, which is an indication of its capacity to reduce nitrate to nitrite when driven with electricity. The 2nd filter or "reactor" was "setup" with 2 ml NiR/NoR/azure-A GDE in a cloth reservoir. The NiR/NoR/azure-A GDE preparation was made on February 5, 1995, by pumping a solution containing both enzymes in a partially purified form over the activated GDE, without a coupling agent added, at 4°C. NiR and NoR assays of the enzyme preparation before and after the column indicated that ∼90% of the enzyme activities were bound. Subsequently, azure A was pumped over the column at 4°C and the binding of the dye was demonstrated by showing that the matrix had about equal activity when assayed for NiR activity with either methyl viologen/dithionite or dithionite only; the activities was 0.7 unit NiR activity/ml GDE matrix (1 unit = 1 µmole nitrite reduced per minute) and 0.2 unit NoR activity/ml GDE matrix (1 unit = 1 µmole benzyl viologen oxidized per minute; NoR activity estimated via net binding of enzyme activity rather than directly with the GDE bound enzyme). The NiR/NoR/azure-A GDE was found to have an activity for NiR of 0.23 unit/ml GDE matrix on March 12, 1995, which was the last day of the prototype water processing experiments. The NaR/azure-A GDE was found to have an activity of 0.53 units/ml GDE matrix on March 12, 1995. Thus, while the GDE matrices had lost some activity for the immobilized enzymes during the month over which the water processing experiments were carried out, the activity loss was probably not more than half of the total available at the beginning of the experiments (for NaR it appears to be greater but this matrix was prepared 1 month prior to the beginning of the experiments and much of the loss of activity of NaR gels found during the first few days after the immobilization, after which it is rather stable).

Finally, it was found that if the GDE matrices were ground in a mortar and pestle prior to packing into the cloth reservoirs, it was a more effective catalyst. This was demonstrated in test tube based assays for NiR activity of NiR/NoR/azure-A GDE. Since this procedure made it easier to load the cloth reservoirs, it was used for preparation of the GDE matrices for most of the experiments described below.

### (2). Deionized Water containing Nitrate and Phosphate

A series of initial experiments were done to establish the working parameters of the system. Deionized water buffered to pH 7.0 with low concentrations of sodium/potassium phosphate (5 to 15 mM) containing about 10 ppm nitrate-N as KNO₃ was used to test the system. With the two reactor system (one reactor for immobilized NaR/azure-A GDE and one for immobilized NiR/NoR/azure-A GDE), the flow rate was varied and it was found that flow rates in the range of 50 to 100 ml/hour resulted in nitrate removal and no accumulation of significant levels of nitrite. Significant levels of nitrite were considered to be greater than 1 ppm nitrite-N, which is the MCL for nitrite (MCL = maximum contaminant limit set by US Environmental Protection Agency for drinking water). The pH of the effluent from the reactors did not rise significantly in these experiments. To demonstrate the capability of the system to remove nitrate from deionized water in a continuous operation, the following data were collected:

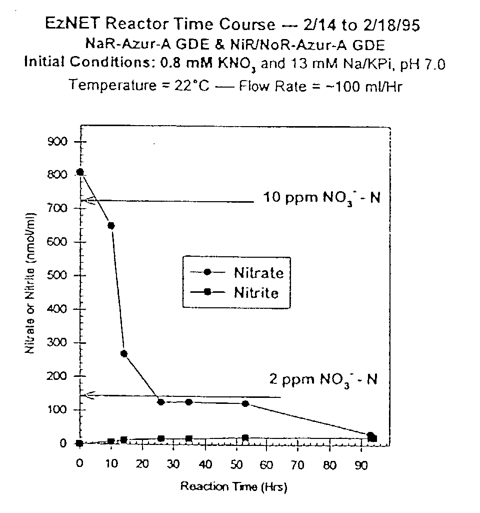

Nitrate removal from deionized water containing nitrate buffer with phosphate to pH 7.0.

The total volume of the water processed was 250 ml which was recirculated through the reactors during this time course of approximately 4 days. It is clear from these results that nitrate is continuously removed and nitrite did not accumulate. The initial nitrate concentration was 11.3 ppm nitrate-N (809 nanomoles nitrate/ml), which was decreased to less than 2 ppm nitrate-N (125 nanomoles nitrate/ml) by 26 hours. Near complete removal of nitrate was achieved by 94 hours when there was only 0.3 ppm nitrate-N (24 nmol nitrate/ml) remaining. There was no nitrite in the water sample initially and by 10 hours it reached about 0.12 ppm nitrite-N and leveled off at about 0.29 ppm nitrite-N. This was well below the MCL level of 1 ppm nitrite-N.

### (3). Lake Linden, Michigan, Tap Water with Nitrate Added

Since the deionized water (containing nitrate with phosphate buffering) experiments demonstrated that the water treatment system is capable of removing large amounts of nitrate without significant accumulation of nitrite, a more realistic performance test was setup using Lake Linden, Michigan, tap water. Lake Linden tap water comes from a deep well and is not chlorinated. Analysis of the water provided by Lake Linden Village indicates the water contains no nitrate or nitrite (analysis provided by Lake Linden City Hall). While the Lake Linden tap water is also low in components found in many municipal water supplies, this tap water probably represents that found in many sites in the U.S. but in no way is it suggested here as being typical of any particular profile of U.S. potable water. Four to five liter batches of tap water were prepared for nitrate removal by stirring overnight and filtration to removal visible particles. The tap water was adjusted from pH 7.8 to ∼7.0 with HCI and KNO₃ added to bring the level of nitrate to about 10 ppm nitrate-N. Then, the tap water spiked with nitrate was pumped into the two-reactor treatment system operating with a 12 volt direct current applied from a DC converter. The voltage drop across the electrodes of the treatment reactors was monitored with a multimeter and it did not change from 12 volts significantly during the experiment. The water was pumped through the reactors in a flow from the reservoir to the receiving container without recycling. Various pumping rates were used during the experiment as indicated below. Nitrate was effectively removed at all pumping rates. In contrast to the deionized water experiments where the highest flow rate for a single pass through the system reactor, which could be used and still obtain the highest effective nitrate removal, was ∼50 ml/hour, nitrate was removed effectively even at higher flow rates (above 100 ml/hour). The explanation for this different behavior may be that the tap water has a higher ion content than the deionized water and, therefore, supported higher current flow in the system. No attempt was made to measure the current within the reactors. Samples were taken from the receiving container at intervals and, along with samples of the initial water placed in the delivery reservoir, were analyzed for nitrate and nitrite content. All samples were analyzed in duplicate and for most samples the analyses were repeated on separate days. Four separate batches of nitrate-spiked Lake Linden tap water were processed over an approximate four day period from March 4 to March 8, 1995. Samples were stored at 4°C until analyzed. The water exiting the reactors was slightly yellow and contained reddish-brown particles. Since the stainless steel electrodes were noted after the experiment was completed to have suffered some corrosion during operation, it was suspected that the electrodes contributed iron ions to the solution which resulted in precipitation of ferric/ferrous chloride during the experiment, but this was not verified.

**Analysis of Samples of Lake Linden, Michigan, Tap Water containing ∼10 ppm Nitrate-N Before and After Processing through Two Treatment system Operating In-Line**

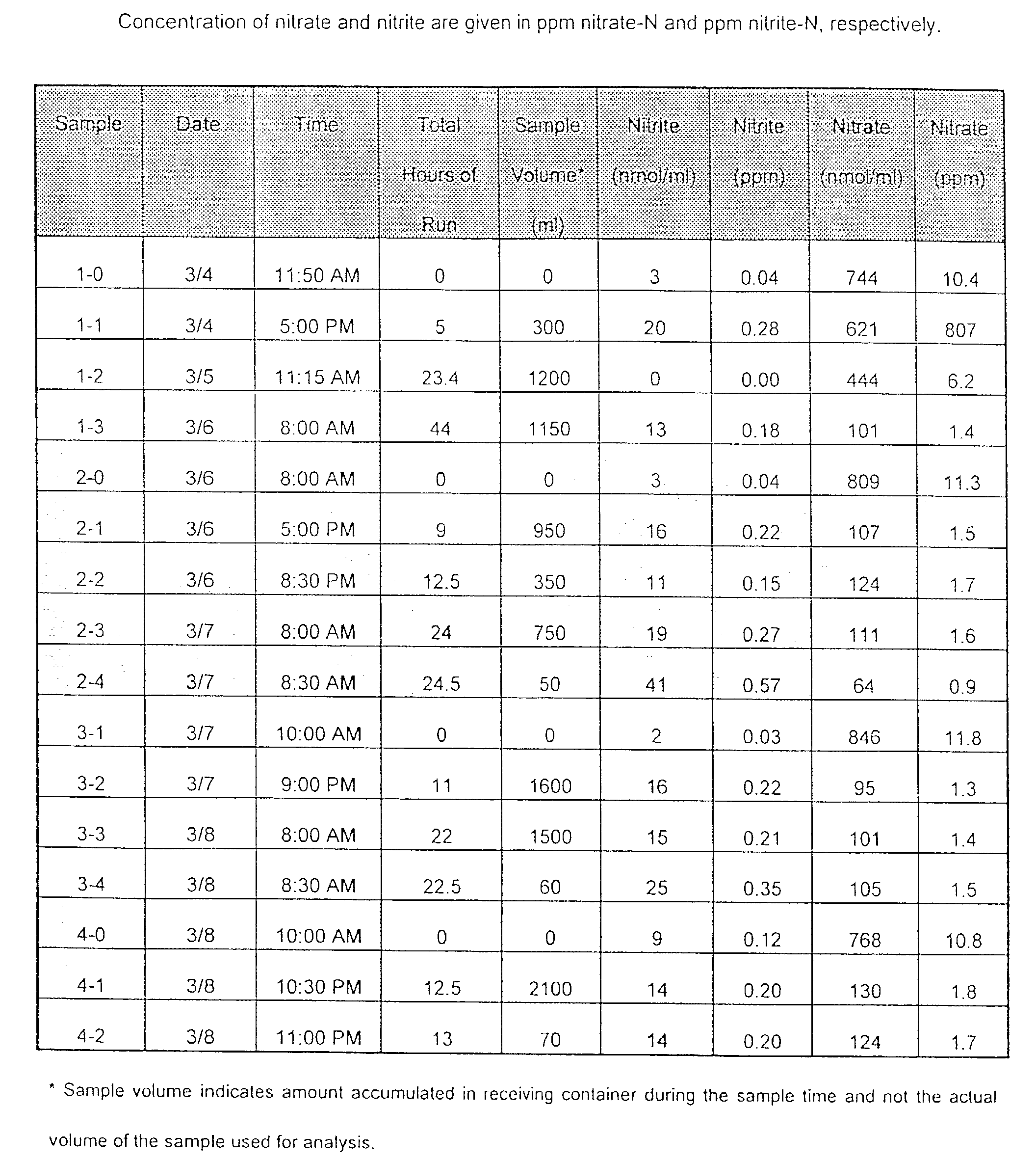

### (4). Summary for Removal of of Nitrate from Nitrate-Spiked Lake Linden Tap Water

Flow rates were calculated by sum of the volume of samples divided by hours of run. Nitrate removal rates were calculated by multiplying the volume of each sample by the difference in the nitrate concentration (initial minus final).

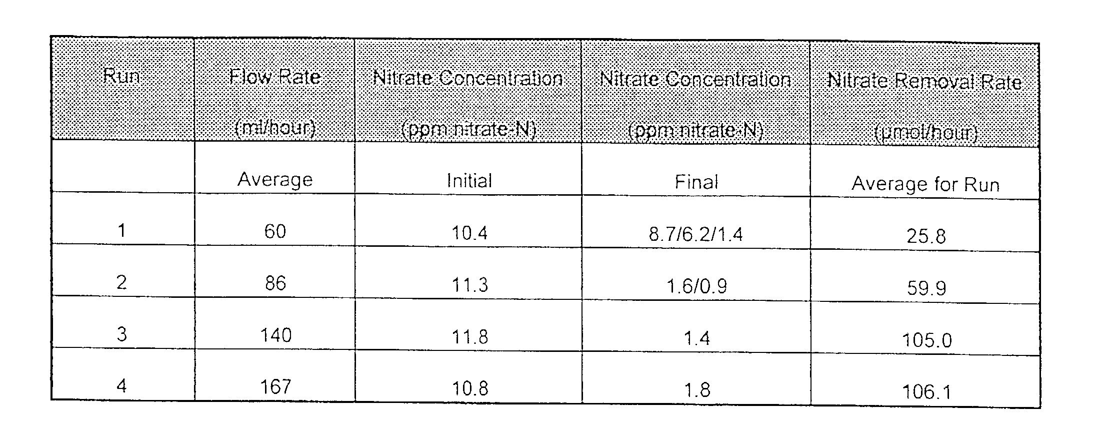

The comparison of tap flow rate in the treatment system with the nitrate removal rate summarized in the table above illustrates the high efficiency of system operation under fairly real conditions much like what may be found in the field.

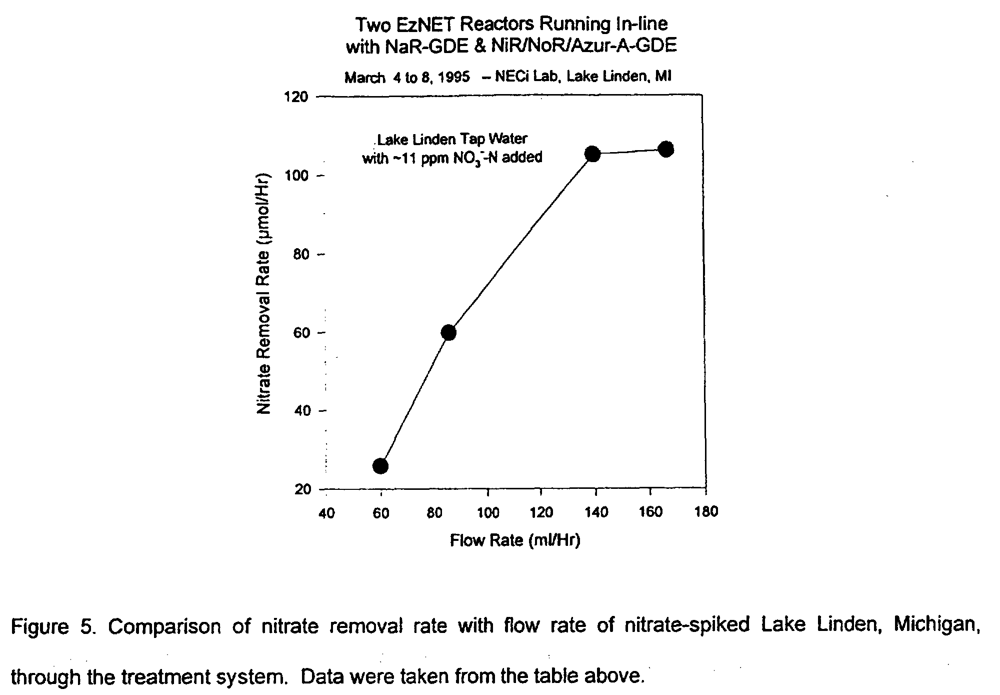

In this experiment, the pH of the tap water was adjusted to about 7.0 before each run was begun. The pH after the run in each case was found to be about 7.4. This is consistent with the reactions catalyzed by the enzymes immobilized in the treatment system, where for each nitrate converted to nitrite, a hydroxide anion is produced. Thus, the rise in pH is expected and the magnitude of increase is within the range expected for the amount of nitrate removed, although this is a bit difficult to predict since the buffer capacity of the tap water was not determined.

### (5). Efficiency of the Enzymes in the Treatment System

Based on the data obtained in the experiments associated with nitrate removal from Lake Linden tap water spiked with nitrate, the catalytic rates for immobilized and electrically driven NaR and NiR can be calculated. Since the maximum rate of nitrate removal was obtained in the last run (see the table in section (e)(4) of this Application), only this data will be used for estimating enzyme catalytic rates. For the 2 ml of immobilized NaR/azure-A GDE matrix used in the treatment system, the NaR activity is 0.9 µmoles nitrate reduced/minute/ml matrix in the treatment system. When NaR/azure-A GDE matrix was assayed at 30°C using the dithionite-only:NaR activity assay, this same immobilized enzyme/dye matrix had 0.5 µmoles nitrite produced/minute/ml matrix. Thus, it appears that NaR/azure-A GDE matrix is more efficient in the treatment system than it is the "solution" assay, despite the lower temperature (ie 22°C) used for the reactor experiment. This is not unexpected since the "solution" assay is static as compared to the flow-through nature of the reactor. For the 2 ml of immobilized NiR/NoR/azure-A GDE matrix used in the treatment system, the rate of nitrite removal would be the virtually same as the rate of nitrate removal calculated for the NaR matrix since nitrite does not accumulate in the system out flow to any significant extent. Thus, immobilized and electrically driven NiR is catalyzing nitrite reduction with an activity of 0.9 µmoles nitrite reduced/minute/ml matrix in the treatment system. When the same NiR/NoR/azure-A GDE matrix was assayed via the "solution" assay 3 days after the end of the tap water experiment, it had an activity of 0.23 µmoles nitrite reduced/minute/ml matrix. Thus, NaR/NoR/azure-A GDE matrix is also more efficient in the treatment system than in the "solution" assay. It is not possible with currently available equipment to determine if the NoR enzyme is active in the treatment system and the NoR activity of the NiR/NoR/azure-A GDE matrix has not been determined.

### (6). Stability of the NaR and NiR in the Treatment System

The final consideration from the data presented here on the performance of the treatment system for removing nitrate from water is the stability of the immobilized enzymes. Rather realistic performance tests were carried out in these experiments in that the treatment system were operated at room temperature (22°C) and even with Lake Linden tap water, which contains many more substances potentially toxic to enzymes than deionized water. The data shown in the tables and figures in section (e)(3) and section (e)(4) attest to the stability of the immobilized enzymes under simulated field conditions. The same preparations of NaR/azure-A and NiR/NoR/azure-A GDE matrices were used in all reactor experiments for which data are presented. In fact, the treatment systems were not disassembled during the 4 days of the experiments. The enzymes appear to be as active at the end of the experiment as in the beginning. Of course, the enzyme will need to function for about 1 month in the field and so these short term experiments need to be extended before the treatment system can be truly field tested. However, the same NiR/NoR/azure-A GDE matrix cloth reservoir was used repeatedly during the first month of performance testing of the treatment system in deionized water containing nitrate and buffered with phosphate with out apparent loss of activity for nitrite removal.

It is to be understood that the invention is not limited to the exact construction or method illustrated and described above, but that various changes and modifications may be made without departing from the spirit and scope of the invention as defined in the following claims.

## Claims

1. A method of obtaining nitrate reductase comprising the steps of:
(a) preparing ZM2,69 monoclonal antibody by loading a certain amount of immunoglobulin G onto a quantity of cyanogen bromide activated Sepharose;
(b) obtaining a plurality of corn plant leaves;
(c) blending said plurality of said corn plant leaves with a certain potassium phosphate solution and a certain quantity of polyvinylpolypyrrolidone, thereby creating a blended mixture; and
(d) mixing said prepared antibody with said blended mixture, thereby obtaining said nitrate reductase from said corn plant leaves.

2. A method to obtain nitrate reductase, said method comprising the steps of:
(a) obtaining a quantity of corn seedlings;
(b) planting said quantity of corn seedlings within a quantity of vermiculite by placing said quantity of said corn seedlings and said quantity of said vermiculite within a container;
(c) storing said container at a temperature of about eighty degrees centigrade for a first certain period of time;
(d) watering said container for said first certain period of time in order to ensure that said vermiculite remains moist;
(e) placing said container under a light for a second certain period of time;
(f) spraying said vermiculite with a certain fertilizer solution, thereby allowing said quantity of said corn seedlings to mature into corn plants having a plurality of leaves;
(g) harvesting said plurality of leaves;
(h) storing said plurality of leaves a temperature of about 80 degrees centigrade;
(i) blending a quantity of said stored leaves with a certain potassium phosphate solution and a certain quantity of polyvinylpolypyrrolidone, thereby creating a blended mixture;
(j) preparing ZM2,69 monoclonal antibody by loading a certain amount of immunoglobulin G onto a quantity of cyanogen bromide activated Sepharose;
(k) mixing said prepared antibody with said blended mixture, thereby obtaining said nitrate reductase.

3. Nitrate reductase obtaining from blending a plurality of corn plant leaves with a certain potassium phosphate solution, a certain quantity of polyvinylpolypyrrolidone and a ZM2,69 monoclonal antibody created by loading a certain amount of immunoglobulin G onto a quantity of cyanogen bromide activated Sepharose.

4. Nitrate reductase obtaining from blending a plurality of corn plant leaves with a certain potassium phosphate solution, a certain quantity of polyvinylpolypyrrolidone and a ZM2,69 monoclonal antibody created by loading a certain amount of immunoglobulin G onto a quantity of cyanogen bromide activated Sepharose and storing at about minus 80 degrees centigrade after transfer to 25 mM Mops, pH 7.0.

5. Nitrate reductase obtaining from blending a plurality of corn plant leaves with a certain potassium phosphate solution, a certain quantity of polyvinylpolypyrrolidone and a ZM2,69 monoclonal antibody created by loading a certain amount of immunoglobulin G onto a quantity of cyanogen bromide activated Sepharose and storing at about minus 20 degrees centigrade after transfer to 25 mM Mops, pH 7.0 and 50% glycerol.

6. Nitrate reductase obtaining from blending a plurality of corn plant leaves with a certain potassium phosphate solution, a certain quantity of polyvinylpolypyrrolidone and a ZM2,69 monoclonal antibody created by loading a certain amount of immunoglobulin G onto a quantity of cyanogen bromide activated Sepharose and storing at about 20 degrees centigrade after transfer to 25 mM Mops, pH 7.0 and 25% sucrose followed by freeze drying to a glass state.

7. A home based nitrate containing compound determination apparatus comprising:
(a) a first test tube containing a quantity of substantially nitrate-free water;
(b) a second test tube containing a quantity of nitrate reductase;
(c) a third test tube containing a quantity of color reagents; and
(d) a pipette used to place a certain sample of water into said first test tube.

8. The apparatus of Claim 7 wherein said nitrate containing compound comprises water.

9. The apparatus of Claim 7 wherein said nitrate containing compound comprises food.

10. The home based testing apparatus of Claim 7 further comprising a color chart.

11. The home based testing apparatus of Claim 7 further comprising a plurality of color tubes.

12. A method for determining the amount and/or concentration of nitrate containing compounds in a target medium is provided. The method, comprise the steps of providing a first group of test tubes; providing a second group of test tubes, each of the first and second group of test tubes being substantially free of nitrate, nitrite, and nitrate containing compounds; placing a substance having a pre-determined amount of nitrate into said first group of test tubes; placing a substance having an unknown amount of nitrate into said second group of test tubes; adding a pre-determined amount of substantially nitrate free de-ionized water to each of said groups of test tubes; adding to and mixing with the substances within each of said group of test tubes a pre-determined amount of buffer, NADH solution, and Nitrate reduction solution; allowing the reaction to proceed in each of said groups of test tubes for about fifteen to about twenty minutes at a temperature of about 30 degrees Centigrade; agitating each of said groups of test tubes for a pre-determined amount of time; adding a certain amount of color reagent to each of said groups of test tubes; placing a certain amount of the substance contained in said first group of test tubes into a cuvette and the absorbance at about 540nm is measured; plotting the absorbance readings received from said first group of test tubes against the concentration of nitrate in each of said first group of test tubes, effective to establish a calibration curve; placing a certain amount of the substance contained in each of said second group of test tubes into a cuvette and measuring the absorbance of each of said substances at about 540nm; and comparing the absorbance measurements associated with each of said test tubes in said second group against said calibration curve, effective to determine the amount of nitrate in each of said second group of test tubes.

13. The method of Claim 12 further comprising the step of obtaining said nitrate reductase from corn leaves.

14. The method of Claim 12 further comprising the step of obtaining said nitrate reductase by mixing a certain amount of ZM2,69 monoclonal antibody with said corn leaves, said monoclonal antibody being prepared by loading a certain amount of immunoglobulin G onto a quantity of cyanogen bromide activated Sepharose.

15. The method of Claim 13 further comprising the steps of blending a quantity of said corn leaves with a certain quantity of polyvinylpolypyrrolidone and a certain potassium phosphate solution.

16. A water treatment system comprising a generally hollow columnar shaped container which communicates with untreated water; a plurality of filters, disposed within said container which one adapted to be in contact with said untreated water; and electrical power source coupled to said plurality of filters and cooperating with said filters to treat said contaminated water.

17. The water treatment system of Claim 16 wherein said untreated water contains an amount of nitrate.

18. The water treatment system of Claim 17 wherein a first of said plurality of filters is effective to convert said nitrate to nitrite.

19. The water treatment system of Claim 18 wherein a second of said plurality of filters is effective to convert said nitrite to nitrous oxide.

20. The water treatment system of Claim 19 wherein a third of said plurality of filters is effective to convert said nitrous oxide to nitrogen gas.

21. The water treatment system of Claim 16 wherein said electrical power source comprises an alternating power source.

22. The water treatment system of Claim 16 wherein said electrical power source comprises a direct current power source.

23. The water treatment system of Claim 16 wherein each of said filters comprises a generally rounded electrode; and a porous bag, adapted to be placed upon said electrode and containing a certain amount of nitrate reductase and electron carrying dye.

24. The water treatment system of Claim 23 wherein said electron carrying dye comprises azure-A.

25. The water treatment system of Claim 23 wherein said nitrate reductase is synthesized according to the following steps:
(a) preparing ZM2,69 monoclonal antibody by loading a certain amount of immunoglobulin G onto a quantity of cyanogen bromide activated Sepharose;
(b) obtaining a plurality of corn plant leaves;
(c) blending said plurality of said corn plant leaves with a certain potassium phosphate solution and a certain quantity of polyvinylpolypyrrolidone, thereby creating a blended mixture; and
(d) mixing said prepared antibody with said blended mixture, thereby obtaining said nitrate reductase from said corn plant leaves.

26. The water treatment system of Claim 24 wherein said nitrate reductase is synthesized according to further steps of:
(a) obtaining a quantity of corn seedlings;
(b) planting said quantity of corn seedlings within a quantity of vermiculite by placing said quantity of said corn seedlings and said quantity of said vermiculite within a container;
(c) storing said container at a temperature of about eighty degrees centigrade for a first certain period of time;
(d) watering said container for said first certain period of time in order to ensure that said vermiculite remains moist;
(e) placing said container under a light for a second certain period of time;
(f) spraying said vermiculite with a certain fertilizer solution, thereby allowing said quantity of said corn seedlings to mature into corn plants having a plurality of leaves;
(g) harvesting said plurality of leaves;
(h) storing said plurality of leaves a temperature of about negative 80 degrees centigrade;
(i) blending a quantity of said stored leaves with a certain potassium phosphate solution and a certain quantity of polyvinylpolypyrrolidone, thereby creating a blended mixture;
(j) preparing ZM2,69 monoclonal antibody by loading a certain amount of immunoglobulin G onto a quantity of cyanogen bromide activated Sepharose;
(k) mixing said prepared antibody with said blended mixture, thereby obtaining said nitrate reductase.

27. A filter which is adapted to treat water, said filter comprising a generally rounded electrode which protrudes from said generally hollow and rounded body; a generally porous bag which contains a quantity of nitrate reductase and electron carrying dye and which is adapted to be placed upon said electrode; and electrical receiving means for receiving a quantity of electrical energy and for coupling said received electrical energy to said electrode effective to allow said nitrate reductase to treat said water.

28. The filter of Claim 27 wherein said electron carrying dye comprises azure-A.

29. The filter of Claim 27 wherein said nitrate reductase is synthesized according to the following steps:
(a) preparing ZM2,69 monoclonal antibody by loading a certain amount of immunoglobulin G onto a quantity of cyanogen bromide activated Sepharose;
(b) obtaining a plurality of corn plant leaves;
(c) blending said plurality of said corn plant leaves with a certain potassium phosphate solution and a certain quantity of polyvinylpolypyrrolidone, thereby creating a blended mixture; and
(d) mixing said prepared antibody with said blended mixture, thereby obtaining said nitrate reductase from said corn plant leaves.

30. The filter of Claim 27 wherein said nitrate reductase is synthesized according to the following steps:
(a) obtaining a quantity of corn seedlings;
(b) planting said quantity of corn seedlings within a quantity of vermiculite by placing said quantity of said corn seedlings and said quantity of said vermiculite within a container;
(c) storing said container at a temperature of about eighty degrees centigrade for a first certain period of time;
(d) watering said container for said first certain period of time in order to ensure that said vermiculite remains moist;
(e) placing said container under a light for a second certain period of time;
(f) spraying said vermiculite with a certain fertilizer solution, thereby allowing said quantity of said corn seedlings to mature into corn plants having a plurality of leaves;
(g) harvesting said plurality of leaves;
(h) storing said plurality of leaves at temperature of about negative 80 degrees centigrade;
(i) blending a quantity of said stored leaves with a certain potassium phosphate solution and a certain quantity of polyvinylpolypyrrolidone, thereby creating a blended mixture;
(j) preparing ZM2,69 monoclonal antibody by loading a certain amount of immunoglobulin G onto a quantity of cyanogen bromide activated Sepharose;
(k) mixing said prepared antibody with said blended mixture, thereby obtaining said nitrate reductase.

31. The filter of Claim 27 wherein said generally hollow and rounded body comprises a column.

32. The filter of Claim 27 wherein said generally hollow and rounded body comprises stainless steel.

33. The filter of Claim 27 wherein said electrode comprises stainless steel.

34. A water treatment system comprising a plurality of filters, positioned in a cascade arrangement within an open ended structure while receiving untreated water, said filters being cooperatively effective to treat said untreated water.

35. The water treatment system of Claim 34 wherein said untreated water contained nitrate.

36. The water treatment system of Claim 35 wherein a first of said plurality of filters converts said nitrate to nitrite.

37. The water treatment system of Claim 36 wherein a second of said plurality of filters converts said nitrite to nitrous oxide.

38. The water treatment system of Claim 37 wherein a third of said plurality of filters convert said nitrous oxide to nitrogen gas.

39. The water treatment system of Claim 34 wherein said plurality of filters comprise three.

40. The water treatment system of Claim 34 wherein said plurality of filters contain a certain amount of nitrate reductase.

41. The water treatment system of Claim 40 wherein said nitrate reductase is made according to the following steps:
(a) preparing ZM2,69 monoclonal antibody by loading a certain amount of immunoglobulin G onto a quantity of cyanogen bromide activated Sepharose;
(b) obtaining a plurality of corn plant leaves;
(c) blending said plurality of said corn plant leaves with a certain potassium phosphate solution and a certain quantity of polyvinylpolypyrrolidone, thereby creating a blended mixture; and
(d) mixing said prepared antibody with said blended mixture, thereby obtaining said nitrate reductase from said corn plant leaves.

42. The water treatment system of Claim 41 wherein said nitrate reductase is made according to the further steps of:
(a) obtaining a quantity of corn seedlings;
(b) planting said quantity of corn seedlings within a quantity of vermiculite by placing said quantity of said corn seedlings and said quantity of said vermiculite within a container;
(c) storing said container at a temperature of about eighty degrees centigrade for a first certain period of time;
(d) watering said container for said first certain period of time in order to ensure that said vermiculite remains moist;
(e) placing said container under a light for a second certain period of time;
(f) spraying said vermiculite with a certain fertilizer solution, thereby allowing said quantity of said corn seedlings to mature into corn plants having a plurality of leaves;
(g) harvesting said plurality of leaves;
(h) storing said plurality of leaves a temperature of about negative 80 degrees centigrade;
(i) blending a quantity of said stored leaves with a certain potassium phosphate solution and a certain quantity of polyvinylpolypyrrolidone, thereby creating a blended mixture;
(j) preparing ZM2,69 monoclonal antibody by loading a certain amount of immunoglobulin G onto a quantity of cyanogen bromide activated Sepharose;
(k) mixing said prepared antibody with said blended mixture, thereby obtaining said nitrate reductase.

43. The water treatment system of Claim 42 wherein said plurality of filters contains an electron carrying dye.

44. The water treatment system of Claim 43 wherein said electron carrying dye comprises azure-A.

45. The water treatment system of Claim 34 wherein said system comprises a source of electrical energy.

46. The water treatment system of Claim 45 wherein said source of electrical energy comprise direct current.

47. The water treatment system of Claim 45 wherein said source of electrical energy comprise alternating current.
